# EUROPEAN PATENT SPECIFICATION

(11) **EP 1 789 139 B1**
(45) Date of publication and mention of the grant of the patent: **21.03.2012**
(21) Application number: 05779369.7
(22) Date of filing: 02.09.2005
(51) Int. Cl.: A61P 31/18, A61K 31/505, A61K 9/20

(54) **FUMARATE OF 4-((4-((4-(2-CYANOETHENYL)-2,6-DIMETHYLPHENYL AMINO -2-PYRIMIDINYL AMINO BENZONITRILE**
FUMARAT AUS 4-((4-((4-(2-CYANOETHENYL)-2,6-DIMETHYLPHENYL AMINO -2-PYRIMIDINYL AMINO BENZONITRIL
HYDROCHLORIDE DE 4-((4-((4-(2-CYANOÉTHÉNYL)-2,6-DIMÉTHYLPHÉNYL AMINO -2-PYRIMIDINYL AMINO BENZONITRILE

(30) Priority: 02.09.2004 MY 0403578; 03.09.2004 WO PCT/EP2004/052028; 25.02.2005 EP 05101447
(43) Date of publication of application: 30.05.2007
(73) Proprietor: Janssen Pharmaceutica NV, 2340 Beerse (BE)
(72) Inventor: STEVENS, Paul Theodoor, Janssen Pharmaceutica N.V., B-2340 Beerse (BE); PEETERS, Jozef, Janssen Pharmaceutica N.V., B-2340 Beerse (BE); VANDECRUYS, Roger P., Janssen Pharmaceutica N.V., B-2340 Beerse (BE); STAPPERS, Alfred E., Janssen PharmaceuticaN.V., B-2340 Beerse (BE); COPMANS, Alex Herman, Janssen Pharmaceutica N.V., B-2340 Beerse (BE)
(74) Representative: UEXKÜLL & STOLBERG
(86) International application number: PCT/EP2005/054341
(87) International publication number: WO 2006/024667

(56) References cited:
- WO-A-03/016306
- WO-A-2004/016581
- WO-A-2005/021001

## Description

The present invention relates to compositions of the fumarate salt of 4-[[4-[[4-(2-cyanoethenyl)-2,6-dimethylphenyl]amino]-2-pyrimidinyl]amino]benzonitrile as claimed.
WO 03/16306 discloses HIV replication inhibiting pyrimidine derivatives among which 4-[[4-[[4-(2-cyanoethenyl)-2,6-dimethylphenyl] amino]-2-pyrimidinyl] amino]-benzonitrile and the pharmaceutically acceptable salts thereof.
WO 04/0162581 disclose processes to prepare 4-[[4-[[4-(2-cyanoethenyl)-2,6-dimethylphenyl]amino]-2-pyrimidinyl]amino]benzonitrile.
4-[[4-[[4-(2-cyanoethenyl)-2,6-dimethylphenyl]amino]-2-pyrimidinyl]amino]-benzonitrile, in particular the E-isomer, has excellent HIV replication inhibiting activity against the wild type of HIV as well as drug and multi drug resistant strains of HIV (i.e. strains which have become resistant to art-known drug(s)). The compound has thus potential to be a good candidate for the development of a medicament for the treatment of HIV infection.

High pharmacological activity, a good pharmacological profile is however not the only factor which determines the drugability of a compound.

A good drug candidate should preferably also be stable chemically as well as physically; should have an acceptable toxicity profile; should have an acceptable bioavailability.

The bioavailability of the compound influences the dose of the compound required for administration in order to reach a therapeutically effective concentration of the compound in the patient. Compounds having a low bioavailability need to be administered in higher doses compared to compounds having a higher bioavailability. Possible consequences of the need for higher doses may comprise : an increased risk to adverse effects; an increase in the size of the dosage form; an increase in the frequency of administration. These factors may influence adherence to antiretroviral therapy. Therapy adherence is one of the most important factors influencing the effectiveness of HIV treatment. Increase in dosing frequency and increase in pill size may lead to reduced therapy adherence and hence reduced therapy effectiveness.

Therefore, when designing a medicament for HIV treatment it is preferable to have an active compound with an acceptable bioavailability.

The bioavailability of a compound intended to be administered orally, is dependent on the compounds solubility in water as well as the compounds permeability (its ability to be absorbed across the intestinal membrane).

A scientific framework for classifying drug substances based on their aqueous solubility and intestinal permeability is the Biopharmaceutics Classification System or BCS. According to the BCS, drug substances are classified as follows:
Class 1: High Solubility - High Permeability
Class 2: Low Solubility - High Permeability
Class 3: High Solubility - Low Permeability
Class 4: Low Solubility - Low Permeability

Compounds with a low solubility or a low permeability (class 2 to 4) may suffer from a low bioavailability when administered orally.

Free base 4-[[4-[[4-(2-cyanoethenyl)-2,6-dimethylphenyl]amino]-2-pyrimidinyl]-amino]benzonitrile can be classified as a BCS class 2 compound and has thus a low solubility in water. 4-[[4-[[4-(2-cyanoethenyl)-2,6-dimethylphenyl]amino]-2-pyrimidinyl]amino]benzonitrile does not only exhibit a low solubility in water, but also in an acidic environment. Consequently, when administered orally in a conventional solid dosage form, a low bioavailability may be expected.

When confronted with a BCS class 2 compound intended for oral administration, a person skilled in pharmaceutical technology would turn to exploring possibilities for improving the compound's solubility, for instance by preparing an appropriate salt. This route was also followed for 4-[[4-[[4-(2-cyanoethenyl)-2,6-dimethylphenyl]-ammo]-2-pyrimidinyl]amino]benzonitrile.

The prepared salts appeared to have only a slight improved solubility in water and in HCl. The prepared salts still belong to BCS class 2. Thus, also for the prepared salts a low bioavailibility could be expected.

Unexpectedly, it has now been found that the fumarate salt (trans CH(COOH)=CH(COOH) of 4-[[4-[[4-(2-cyanoethenyl)-2,6-dimethylphenyl]amino]-2-pyrimidinyl]amino]benzonitrile, in particular its E-isomer, has a significant improved *in vivo* bioavailability compared to the free base. In fact, the present salt administered as a solid dosage form has an *in vivo* bioavailability which is comparable with the bioavailability of the free base administered as an oral PEG 400 solution.

Because of the increased bioavailability *in vivo*, the fumarate salt may be formulated without the need of complex formulation techniques.

The fumarate salt of the present invention was also found to be non-hygroscopic and to be chemically and physically stable in different conditions of humidity and temperatures.

Therefore, the present invention relates to a compound of formula (I), i.e. the fumarate (trans CH(COOH)=CH(COOH) salt of 4-[[4-[[4-(2-cyanoethenyl)-2,6-dimethylphenyl]amino]-2-pyrimidinyl]amino]benzonitrile, a *N*-oxide or a stereochemically isomeric form thereof.

Thus, the present invention relates in particular to a solid pharmaceutical composition for oral administration comprising a pharmaceutically acceptable carrier and as active ingredient a therapeutically effective amount of a compound of formula (I) 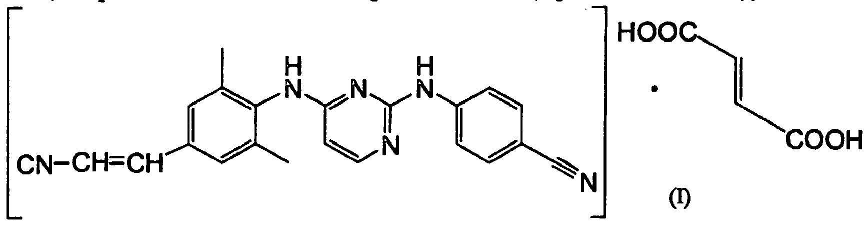 a *N*-oxide or a stereochemically isomeric form thereof, and further comprising a wetting agent.

The *N*-oxide forms of the present compound of formula (I) are meant to comprise the compounds of formula (I) wherein one or several tertiary nitrogen atoms are oxidized to the so-called *N*-oxide.

The term "stereochemically isomeric forms" as used hereinbefore defines all the possible stereoisomeric forms which the compound of formula (I), and the *N*-oxides, or quaternary amines may possess. Unless otherwise mentioned or indicated, the chemical designation of the compound denotes the mixture of all possible stereochemically isomeric forms as well as each of the individual isomeric forms of formula (I) and the *N*-oxides, solvates or quaternary amines substantially free of the other isomers. Stereochemically isomeric forms of the compound of formula (I) are obviously intended to be embraced within the scope of this invention.

The compound of formula (I) may exist in 2 stereochemical configurations at the double bond of the cyanoethenyl chain, i.e. the E (Entgegen) configuration (E-isomer) and the Z (Zusammen) configuration (Z isomer).
The terms E and Z are well known to a person skilled in the art.

A particular embodiment of the compound of formula (I) is the E-isomer, i.e. a compound of formula (I-a)

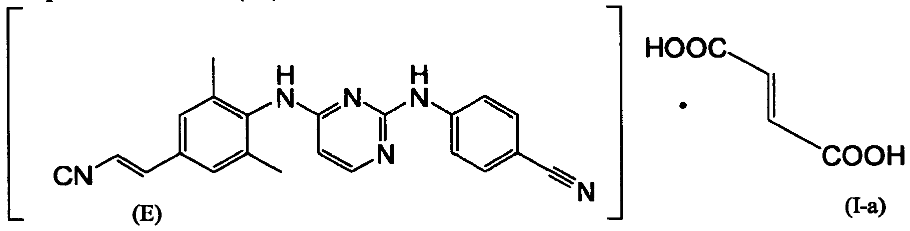

Another particular embodiment of the compound of formula (I) is the Z-isomer, i.e. a compound of formula (I-b)

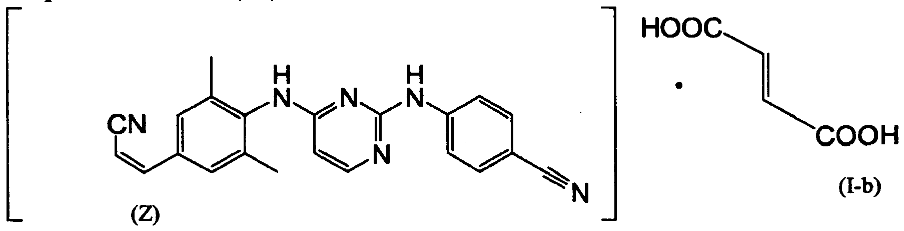

Whenever reference is made herein to the E-isomer, the pure E-isomer or any isomeric mixture of the E- and the Z-isomers wherein the E-isomer is predominantly present is meant, i.e. an isomeric mixture containing more than 50% or in particular more than 80% of the E-isomer, or even more in particular more than 90% of the E-isomer. Of particular interest is the E-isomer substantially free of the Z-isomer. Substantially free in this context refers to E-Z-mixtures with no or almost no Z-isomer, e.g. isomeric mixtures containing as much as 90%, in particular 95% or even 98% or 99% of the E-isomer.

Whenever reference is made herein to the Z-isomer, the pure Z-isomer or any isomeric mixture of the Z- and the E-isomers wherein the Z-isomer is predominantly present is meant, i.e. an isomeric mixture containing more than 50% or in particular more than 80% of the Z-isomer, or even more in particular more than 90% of the Z-isomer. Of particular interest is the Z-isomer substantially free of the E-isomer. Substantially free in this context refers to E-Z-mixtures with no or almost no E-isomer, e.g. isomeric mixtures containing as much as 90%, in particular 95% or even 98% or 99% of the Z-isomer.

Polymorphic forms of the present salts also fall within the ambit of the present invention.

Polymorphic forms of pharmaceutical compounds may be of interest to those involved in the development of a suitable dosage form because if the polymorphic form is not held constant during clinical and stability studies, the exact dosage used or measured may not be comparable from one lot to the next. Once a pharmaceutical compound is produced for use, it is important to recognize the polymorphic form delivered in each dosage form to assure that the production process use the same form and that the same amount of drug is included in each dosage. Therefore, it is imperative to assure that either a single polymorphic form or some known combination of polymorphic forms is present. In addition, certain polymorphic forms may exhibit enhanced thermodynamic stability and may be more suitable than other polymorpholic forms for inclusion in pharmaceutical formulations. As used herein, a polymorphic form of a compound of the invention is the same chemical entity, but in a different crystalline arrangement.

Solvent addition forms (solvates) which the salts of the present invention are able to form also fall within the ambit of the present invention. Examples of such forms are e.g. hydrates, alcoholates and the like. Solvates are herein also referred to as pseudopolymorphic forms. Preferred is an anhydric salt.

Whenever used hereinafter, the term "compound of formula (I), (I-a) or (I-b)" is meant to also include the *N*-oxide forms, the stereochemically isomeric forms and the polymorphic or pseudopolymorphic forms. Of special interest is a stereochemically pure form of a compound of formula (I). A preferred compound of formula (I) is a compound of formula (I-a).

The compounds of formula (I), (I-a) or (I-b) can be prepared by reacting the corresponding free base with fumaric acid in the presence of a suitable solvent, such as for example a suitable acid, e.g. acetic acid.

The compounds of formula (I), (I-a) or (I-b) have antiretroviral activity. They are able to inhibit the replication of HIV, in particular HIV-1. HIV (Human Immunodeficiency Virus) is the aetiological agent of Acquired Immune Deficiency Syndrome (AIDS) in humans. The HIV virus preferentially infects human T-4 cells and destroys them or changes their normal function, particularly the coordination of the immune system. As a result, an infected patient has an ever decreasing number of T-4 cells, which moreover behave abnormally. Hence, the immunological defense system is unable to combat infections and neoplasms and the HIV infected subject usually dies by opportunistic infections such as pneumonia, or by cancers. Other conditions associated with HIV infection include thrombocytopaenia, Kaposi's sarcoma and infection of the central nervous system characterized by progressive demyelination, resulting in dementia and symptoms such as, progressive dysarthria, ataxia and disorientation. HIV infection further has also been associated with peripheral neuropathy, progressive generalized lymphadenopathy (PGL) and AIDS-related complex (ARC).

The present compounds also show activity against drug and multidrug resistant HIV strains, in particular drug and multidrug resistant HIV-1 strains, more in particular the present compounds show activity against HIV strains, especially HIV-1 strains, that have acquired resistance to one or more art-known non-nucleoside reverse transcriptase inhibitors. Art-known non-nucleoside reverse transcriptase inhibitors are those non-nucleoside reverse transcriptase inhibitors other than the present compounds and in particular commercial non-nucleoside reverse transcriptase inhibitors.

The HIV replication inhibiting activity of 4-[[4-[[4-(2-cyanoethenyl)-2,6-dimethylphenyl]amino]-2-pyrimidinyl]amino]benzonitrile is described in WO 03/16306.

Due to their antiretroviral properties, particularly their anti-HIV properties, especially their HIV-1 replication inhibiting activity, the present compounds are useful in the treatment of individuals infected by HIV and for the prophylaxis of these infections. In general, the compounds of the present invention may be useful in the treatment of warm-blooded mammals infected with viruses whose existence is mediated by, or depends upon, the enzyme reverse transcriptase. Conditions which may be prevented or treated with the compounds of the present invention, especially conditions associated with HIV and other pathogenic retroviruses, include AIDS, AIDS-related complex (ARC), progressive generalized lymphadenopathy (PGL), as well as chronic Central Nervous System diseases caused by retroviruses, such as, for example HIV mediated dementia and multiple sclerosis
Therefrom, the compounds of formula (I), (I-a) or (I-b) can be used as a medicine.

The composition of the present invention may therefore be used as medicines against above-mentioned conditions. Said use as a medicine comprises the administration to HIV-infected subjects of an amount effective to combat the conditions associated with HIV and other pathogenic retroviruses, especially HIV-1. In particular, the present composition may be used in the manufacture of a medicament for the treatment or the prevention of HIV infection, preferably for the treatment of HIV infection.

In view of the utility of the present compounds, there is also provided a composition for use in treating mammals, including humans, suffering from or a method of preventing warm-blooded mammals, including humans, to suffer from viral infections, especially HIV infections. Said method comprises the oral administration, of an effective amount of a composition of the present invention to mammals including humans.

Due to the higher bioavailability of the present compounds compared to the corresponding free base, therapeutic effective plasma levels may be obtained by administering a pharmaceutical composition comprising a lower amount of the salt compared to what would be needed of the corresponding free base.
Therefore, the size of the pharmaceutical composition may be reduced or the frequency of dosing may be reduced.

In particular, the present invention also relates to a pharmaceutical composition according to claim 1 comprising a pharmaceutically acceptable carrier and as active ingredient a therapeutically effective amount of a compound of formula (I), (I-a) or (I-b) provided that the composition does not contain one or more nucleoside reverse transcriptase inhibitors and/or one or more nucleotide reverse transcriptase inhibitors.

Also disclosed is that compounds of formula (I), (I-a) or (I-b) may be formulated into various pharmaceutical compositions for administration purposes. As appropriate compositions there may be cited all compositions usually employed for systemically administering drugs. To prepare the pharmaceutical compositions of this invention, an effective amount of the compound of formula (I), (I-a) or (I-b) as the active ingredient is combined in intimate admixture with a pharmaceutically acceptable carrier, which carrier may take a wide variety of forms depending on the form of preparation desired for administration. These pharmaceutical compositions are desirable in unitary dosage form suitable for administration orally. For example, in preparing the compositions in oral dosage form, any of the usual pharmaceutical media may be employed such as, for example, solid carriers such as starches, sugars, kaolin, diluents, lubricants, binders, disintegrating agents and the like in the case of powders, pills, capsules, and tablets. Because of their ease in administration, tablets and capsules represent the most advantageous oral unit dosage forms, in which case solid pharmaceutical carriers are obviously employed. Also included are solid form preparations, which are intended to be converted, shortly before use, to liquid form preparations. The salts of the present invention may also be administered via inhalation or insufflation by means of methods and formulations employed in the art for administration via this way. Thus, in general the salts of the present invention may be administered to the lungs in the form of dry powder. Any system developed for the delivery of dry powders via oral inhalation are suitable for the administration of the present compounds.

WO 2004/069812 describes the ability of pyrimidine derivatives among which 4-[[4-[[4-(2-cyanoethenyl)-2,6-dimethylphenyl]-amino]-2-pyrimidinyl]amino]benzonitrile and pharmaceutically acceptable salts thereof, to prevent HIV infection via sexual intercourse or related intimate contact between partners. In order to increase the residence time of such pharmaceutical composition at the site of administration, it may be advantageous to include in the composition a bioadhesive, in particular a bioadhesive polymer. A bioadhesive may be defined as a material that adheres to a live biological surface such as for example a mucus membrane or skin tissue.

Thus, the present invention also relates to a pharmaceutical composition comprising a pharmaceutically acceptable carrier and as active ingredient an effective amount of a compound of formula (I), (I-a) or (I-b) characterized in that the pharmaceutical composition is bioadhesive to the site of application. In particular, the present invention also relates to a pharmaceutical composition comprising a pharmaceutically acceptable carrier and as active ingredient an effective amount of a compound of formula (I), (I-a) or (I-b) characterized in that the pharmaceutical composition is bioadhesive to the site of application provided that the composition does not contain one or more nucleoside reverse transcriptase inhibitors and/or one or more nucleotide reverse transcriptase inhibitors. Preferably, the site of application is the mouth.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, powder packets, wafers, and the like, and segregated multiples thereof.

The exact dosage and frequency of administration depends on the particular condition being treated, the severity of the condition being treated, the age, weight, sex, extent of disorder and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention.

The pharmaceutical compositions of the present invention can be administered at any time of the day independently of the food taken in by the subject. Preferably, the present compositions are administered to fed subjects.

The present invention concerns an oral pharmaceutical composition, i.e. a pharmaceutical composition suitable for oral administration, comprising a pharmaceutically acceptable carrier and as active ingredient a therapeutically effective amount of a compound of formula (I), (I-a) or (I-b); in particular a pharmaceutical composition suitable for oral administration, comprising a pharmaceutically acceptable carrier and as active ingredient a therapeutically effective amount of a compound of formula (I), (I-a) or (I-b) provided that the composition does not contain one or more nucleoside reverse transcriptase inhibitors and/or one or more nucleotide reverse transcriptase inhibitors.

In particular, the oral pharmaceutical composition is a solid oral pharmaceutical composition, more in particular a tablet or a capsule, even more in particular a tablet. A tablet according to the present invention may be formulated as a once daily tablet.

Preferably, the pharmaceutical compositions of the present invention contain those quantities of a compound of formula (I), (I-a) or (I-b) equivalent to from about 5 to about 500 mg of the corresponding free base 4-[[4-[[4-(2-cyanoethenyl)-2,6-dimethylphenyl]amino]-2-pyrimidinyl]amino]benzonitrile, its E or Z isomer, more preferably from about 10 mg to about 250 mg of the corresponding free base, even more preferably from about 20 mg to about 200 mg of the corresponding free base. Preferably, the present pharmaceutical compositions contain those quantities of a compound of formula (I), (I-a) or (I-b) equivalent to 25 mg, 50 mg, 75 mg, 100 mg or 150 mg of the corresponding free base (base equivalent).

As used hereinbefore or hereinafter, the term "about" in relation to a numerical value x means, for example, x ±10 %.

The particle size of the compound of formule (I), (I-a) or (I-b) preferably is less than 50 µm, more preferably less than 25 µm, even more preferably less than 20 µm. Further preferred is a particle size of about 15 µm or less, or about 12 µm or less, or about 10 µm or less, or about 5 µm or less. Most preferably, the particle size ranges between about 0.2 and about 15 µm or between about 0.2 and about 10 µm.

The pharmaceutical compositions of the present invention comprise a wetting agent.

As for the wetting agent in the compositions of the invention, there may be used any of the physiologically tolerable wetting agent suitable for use in a pharmaceutical composition.

It is well-known in the art that a wetting agent is an amphiphilic compound; it contains polar, hydrophilic moieties as well as non-polar, hydrophobic moieties.

The terms "hydrophilic" or "hydrophobic" are relative terms.

The relative hydrophilicity or hydrophobicity of a wetting agent may be expressed by its hydrophilic-lipophilic balance value ("HLB value). Wetting agents with a lower HLB value are catagorized as being "hydrophobic" wetting agents whereas wetting agents with a higher HLB value are catagorized as being "hydrophilic" wetting agents. As a rule of thumb, wetting agents having a HLB value greater than about 10 are generally considered as being hydrophilic wetting agents; wetting agents having a HLB value lower than about 10 are generally considered as being hydrophobic wetting agents.

The present compositions preferably comprise a hydrophilic wetting agent. It should be appreciated that the HLB value of a wetting agent is only a rough guide to indicate the hydrophilicity/hydrophobicity of a wetting agent. The HLB value of a particular wetting agent may vary depending upon the method used to determine the HLB value; may vary depending on its commercial source; is subject to batch to batch variability. A person skilled in the art can readily identify hydrophilic wetting agents suitable for use in the pharmaceutical compositions of the present invention.

The wetting agent of the present invention can be an anionic, a cationic, a zwitterionic or a non-ionic wetting agent, the latter being preferred. The wetting agent of the present invention can also be a mixture of two or more wetting agents.

Suitable wetting agents for use in the compositions of the present invention are listed below. In the present compositions, also mixtures of wetting agents may be used.

Suitable wetting agents which may be used in the present invention comprise :
a) Polyethylene glycol fatty acid monoesters comprising esters of lauric acid, oleic acid, stearic acid, ricinoic acid and the like with PEG 6, 7, 8, 9, 10, 12, 15, 20, 25, 30, 32, 40, 45, 50, 55, 100, 200, 300, 400, 600 and the like, for instance PEG-6 laurate or stearate, PEG-7 oleate or laurate, PEG-8 laurate or oleate or stearate, PEG-9 oleate or stearate, PEG-10 laurate or oleate or stearate, PEG-12 laurate or oleate or stearate or ricinoleate, PEG-15 stearate or oleate, PEG-20 laurate or oleate or stearate, PEG-25 stearate, PEG-32 laurate or oleate or stearate, PEG-30 stearate, PEG-40 laurate or oleate or stearate, PEG-45 stearate, PEG-50 stearate, PEG-55 stearate, PEG-100 oleate or stearate, PEG-200 oleate, PEG-400 oleate, PEG-600 oleate; (the wetting agents belonging to this group are for instance known as Cithrol, Algon, Kessco, Lauridac, Mapeg, Cremophor, Emulgante, Nikkol, Myrj, Crodet, Albunol, Lactomul)
b) Polyethylene glycol fatty acid diesters comprising diesters of lauric acid, stearic acid, palmic acid, oleic acid and the like with PEG-8, 10, 12, 20, 32, 400 and the like, for instance PEG-8 dilaurate or distearate, PEG-10 dipalmitate, PEG-12 dilaurate or distearate or dioleate, PEG-20 dilaurate or distearate or dioleatePEG-32 dilaurate or distearate or dioleate, PEG-400 dioleate or distearate; (the wetting agents belonging to this group are for instance known as Mapeg, Polyalso, Kessco, Cithrol)
c) Polyethylene glycol fatty acid mono-and diester mixtures such as for example PEG 4-150 mono and dilaurate, PEG 4-150 mono and dioleate, PEG 4-150 mono and distearate and the like; (the wetting agents belonging to this group are for instance known as Kessco)
d) Polyethylene glycol glycerol fatty acid esters such as for instance PEG-20 glyceryl laurate or glyceryl stearate or glyceryl oleate, PEG-30 glyceryl laurate or glyceryl oleate, PEG-15 glyceryl laurate, PEG-40 glyceryl laurate and the like; (the wetting agents belonging to this group are for instance known as Tagat, Glycerox L, Capmul),
e) Alcohol-oil transesterification products comprising esters of alcohols or polyalcohols such as glycerol, propylene glycol, ethylene glycol, polyethylene glycol, sorbitol, pentaerythritol and the like with natural and/or hydrogenated oils or oil-soluble vitamins such as castor oil, hydrogenated castor oil, vitamin A, vitamin D, vitamin E, vitamin K, an edible vegetable oil e.g. corn oil, olive oil, peanut oil, palm kernel oil, apricot kernel oil, almond oil and the like, such as PEG-20 castor oil or hydrogenated castor oil or corn glycerides or almond glycerides, PEG-23 castor oil , PEG-25 hydrogenated castor oil or trioleate, PEG-35 castor oil, PEG-30 castor oil or hydrogenated castor oil, PEG-38 castor oil, PEG-40 castor oil or hydrogenated castor oil or palm kernel oil, PEG-45 hydrogenated castor oil, PEG-50 castor oil or hydrogenated castor oil, PEG-56 castor oil, PEG-60 castor oil or hydrogenated castor oil or corn glycerides or almond glycerides, PEG-80 hydrogenated castor oil, PEG-100 castor oil or hydrogenated castor oil, PEG-200 castor oil, PEG-8 caprylic/capric glycerides, PEG-6 caprylic/capric glycerides, lauroyl macrogol-32 glyceride, stearoyl macrogol glyceride, tocopheryl PEG-1000 succinate (TPGS); (the wetting agents belonging to this group are for instance known as Emalex, Cremophor, Emulgante, Eumulgin, Nikkol, Thornley, Simulsol, Cerex, Crovol, Labrasol, Softigen, Gelucire, Vitamin E TPGS),
f) polyglycerized fatty acids comprising polyglycerol esters of fatty acids such as for instance polyglyceryl-10 laurate or oleate or stearate, polyglyceryl-10 mono and dioleate, polyglyceryl polyricinoleate and the like; (the wetting agents belonging to this group are for instance known as Nikkol Decaglyn, Caprol or Polymuls)
g) Sterol derivatives comprising polyethylene glycol derivatives of sterol such as PEG-24 cholesterol ether, PEG-30 cholestanol, PEG-25 phyto sterol, PEG-30 soya sterol and the like; (the wetting agents belonging to this group are for instance known as Solulan™ or Nikkol BPSH)
h) Polyethylene glycol sorbitan fatty acid esters such as for example PEG-10 sorbitan laurate, PEG-20 sorbitan monolaurate or sorbitan tristearate or sorbitan monooleate or sorbitan trioleate or sorbitan monoisostearate or sorbitan monopalmiate or sorbitan monostearate, PEG-4 sorbitan monolaurate, PEG-5 sorbitan monooleate, PEG-6 sorbitan monooleate or sorbitan monolaurate or sorbitan monostearate, PEG-8 sorbitan monostearate, PEG-30 sorbitan tetraoleate, PEG-40 sorbitan oleate or sorbitan tetraoleate, PEG-60 sorbitan tetrastearate, PEG-80 sorbitan monolaurate, PEG sorbitol hexaoleate (Atlas G-1086) and the like; (the wetting agents belonging to this group are for instance known as Liposorb, Tween, Dacol MSS, Nikkol, Emalex, Atlas)
i) Polyethylene glycol alkyl ethers such as for instance PEG-10 oleyl ether or cetyl ether or stearyl ether, PEG-20 oleyl ether or cetyl ether or stearyl ether, PEG-9 lauryl ether, PEG-23 lauryl ether (laureth-23), PEG-100 stearyl ether and the like; (the wetting agents belonging to this group are for instance known as Volpo, Brij)
j) Sugar esters such as for instance sucrose distearate/monostearate, sucrose monostearate or monopalmitate or monolaurate and the like; (the wetting agents belonging to this group are for instance known as Sucro ester, Crodesta, Saccharose monolaurate)
k) Polyethylene glycol alkyl phenols such as for instance PEG-10-100 nonyl phenol (Triton X series), PEG-15-100 ocyl phenol ether (Triton N series) and the like;
l) Polyoxyethylene-polyoxypropylene block copolymers (poloxamers) such as for instance poloxamer 108, poloxamer 188, poloxamer 237, poloxamer 288 and the like; (the wetting agents belonging to this group are for instance known as Synperonic PE, Pluronic, Emkalyx, Lutrol™, Supronic, Monolan, Pluracare, Plurodac)
m) ionic wetting agents including cationic, anionic and zwitterionic surfactans such as the fatty acid salts e.g. sodium oleate, sodium lauryl sulfate, sodium lauryl sarcosinate, sodium dioctyl sulfosuccinate, sodium myristate, sodium palmitate, sodium state, sodium ricinoleate and the like; such as bile salts e.g. sodium cholate, sodium taurocholate, sodium glycocholate and the like; such as phospholipids e.g. egg/soy lecithin, hydroxylated lecithin, lysophosphatidylcholine, phosphatidylcholine, phosphatidyl ethanolamine, phosphatidyl glycerol, phosphatidyl serine and the like; such as phosphoric acid esters e.g. diethanolammonium polyoxyethylene-10 oleyl ether phosphate, esterification products of fatty alcohols or fatty alcohol ethoxylates with phosphoric acid or anhydride; such as carboxylates e.g. succinylated monoglycerides, sodium stearyl fumarate, stearoyl propylene glycol hydrogen succinate, mono/diacetylated tartaric acid esters of mono-and diglycerides, citric acid esters of mono-and diglycerides, glyceryl-lacto esters of fatty acids, lactylic esters of fatty acids, calcium/sodium stearoyl-2-lactylate, calcium/sodium stearoyl lactylate, alginate salts, propylene glycol alginate, ether carboxylates and the like; such as sulfates and sulfonates e.g. ethoxylated alkyl sulfates, alkyl benzene sulfates, alpha-olefin sulfonates, acyl isethionates, acyl taurates, alkyl glyceryl ether sulfonates, octyl sulfosuccinate disodium, disodium undecyleneamido-MEA-sulfosuccinate and the like; such as cationic wetting agents e.g. hexadecyl triammonium bromide, decyl trimethyl ammonium bromide, cetyl trimethyl ammonium bromide, dodecyl ammonium chloride, alkyl benzyldimethylammonium salts, diisobutyl phenoxyethoxydimethyl benzylammonium salts, alkylpyridinium salts, betaines (lauryl betaine), ethoxylated amines (polyoxyethylene-15 coconut amine) and the like.

When in the above list of suitable wetting agents, different possibilities are listed such as for example PEG-20 oleyl ether or cetyl ether or stearyl ether, this means that PEG-20 oleyl ether and PEG-20 cetyl ether and PEG-20 stearyl ether are intended. Thus for instance PEG-20 castor oil or hydrogenated castor oil or corn glycerides or almond glycerides has to be read as PEG-20 castor oil and PEG-20 hydrogenated castor oil and PEG-20 corn glycerides and PEG-20 almond glycerides.

Preferred wetting agents in the present compositions are sodium lauryl sulfate, sodium dioctyl sulfosuccinate, or those wetting agents belonging to the group of the polyethylene glycol sorbitan fatty acid esters, such as wetting agents known as Tween, e.g. Tween 20, 60, 80. Most preferred, the wetting agent is Tween 20.

In the compositions of the invention, the wetting agent is preferably present at a concentration from about 0.01 to about 5% by weight relative to the total weight of the composition, preferably from about 0.1 to about 3 % by weight, more preferably from about 0.1 to about 1 % by weight.

The quantity of wetting agent used in the present compositions may depend on the amount of the compound of formula (I), (I-a) or (I-b) present in the composition or on the particle size of the compound of formula (I), (I-a) or (I-b). A higher amount or a smaller particle size may require more wetting agent.

Regarding the solid oral pharmaceutical composition according to the present invention, such as a tablet or a capsule, the composition may also further contain an organic polymer.

The organic polymer may be used as a binder during the manufacture of the composition.

The organic polymer used in the compositions of the invention may be any of the physiologically tolerable water soluble synthetic, semi-synthetic or non-synthetic organic polymers.

Thus for example the polymer may be a natural polymer such as a polysaccharide or polypeptide or a derivative thereof, or a synthetic polymer such as a polyalkylene oxide (e.g. PEG), polyacrylate, polyvinylpyrrolidone, etc. Mixed polymers, e.g. block copolymers and glycopeptides may of course also be used.

The polymer conveniently has a molecular weight in the range 500D to 2 MD, and conveniently has an apparent viscosity of 1 to 15,000 mPa.s when in a 2% aqueous solution at 20°C. For example, the water-soluble polymer can be selected from the group comprising
- alkylcelluloses such as methylcellulose,
- hydroxyakylcelluloses such as hydroxymethylcellulose, hydroxyethylcellulose, hydroxypropylcellulose and hydroxybutylcellulose,
- hydroxyalkyl alkylcelluloses such as hydroxyethyl methylcellulose and hydroxypropyl methylcellulose,
- carboxyalkylcelluloses such as carboxymethylcellulose,
- alkali metal salts of carboxyalkylcelluloses such as sodium carboxymethylcellulose,
- carboxyalkylalkylcelluloses such as carboxymethylethylcellulose,
- carboxyalkylcellulose esters,
- starches,
- pectins such as sodium carboxymethylamylopectin,
- chitin derivates such as chitosan,
- heparin and heparinoids,
- polysaccharides such as alginic acid, alkali metal and ammonium salts thereof, carrageenans, galactomannans, tragacanth, agar-agar, gum arabic, guargum and xanthan gum,
- polyacrylic acids and the salts thereof,
- polymethacrylic acids and the salts thereof, methacrylate copolymers,
- polyvinylalcohol,
- polyvinylpyrrolidone, copolymers of polyvinylpyrrolidone with vinyl acetate,
- polyalkylene oxides such as polyethylene oxide and polypropylene oxide and copolymers of ethylene oxide and propylene oxide, e.g. poloxamers and poloxamines.

Non-enumerated polymers which are pharmaceutically acceptable and have appropriate physico-chemical properties as defined hereinbefore are equally suited for preparing compositions according to the present invention.

Preferably the organic polymer is starch, polyvinylpyrrolidone or a cellulose ether, e.g. PVP K29-32, PVP K90, methyl cellulose, hydroxypropylcellulose, hydroxyethyl methylcellulose, or hydroxypropyl methylcellulose (HPMC).

Said HPMC contains sufficient hydroxypropyl and methoxy groups to render it water-soluble. HPMC having a methoxy degree of substitution from about 0.8 to about 2.5 and a hydroxypropyl molar substitution from about 0.05 to about 3.0 are generally water-soluble. Methoxy degree of substitution refers to the average number of methyl ether groups present per anhydroglucose unit of the cellulose molecule. Hydroxypropyl molar substitution refers to the average number of moles of propylene oxide which have reacted with each anhydroglucose unit of the cellulose molecule. A preferred HPMC is hypromellose 2910 15 mPa.s or hypromellose 2910 5mPa.s, especially hypromellose 2910 15 mPa.s. Hydroxypropyl methylcellulose is the United States Adopted Name for hypromellose (see Martindale, The Extra Pharmacopoeia, 29th edition, page 1435). In the four digit number "2910", the first two digits represent the approximate percentage of methoxyl groups and the third and fourth digits the approximate percentage composition of hydroxypropoxyl groups ; 15 mPa.s or 5 mPa.s is a value indicative of the apparent viscosity of a 2 % aqueous solution at 20°C.

In the compositions of the invention the organic polymer may conveniently be present up to about 10% by weight, preferably from about 0.1 to about 5%, more preferably from about 0.5 to about 3% by weight (relative to the total weight of the composition).

Regarding the solid oral pharmaceutical composition according to the present invention, such as a tablet or a capsule, the composition may also further contain a diluent and/or a glidant.

Pharmaceutical acceptable diluents comprise calcium carbonate, dibasic calcium phosphate, dibasic calcium phosphate dihydrate, tribasic calcium phosphate, calcium sulfate, microcrystalline cellulose including silicified microcrystalline cellulose, powdered cellulose, dextrates, dextrin, dextrose excipient, fructose, kaolin, lactitol, lactose anhydrous, lactose monohydrate, mannitol, sorbitol, starch, pregelatinized starch, sodium chloride, sucrose, compressible sugar, confectioner's sugar, a spray-dried mixture of lactose monohydrate and microcrystalline cellulose (75:25), commercially available as Microcelac^{®}, a co-processed spray-dried mixture of microcrystalline cellulose and colloidal silicon dioxide (98:2), commercially available as Prosolv^{®}. Preferred is lactose monohydrate, microcrystalline cellulose or silicified microcrystalline cellulose.

Pharmaceutically acceptable glidants comprise talc, colloidal silicon dioxide, starch. magnesium stearate. Preferred is colloidal silicon dioxide.

In case of a tablet, the composition may also further comprise a disintegrant and a lubricant.

Pharmaceutically acceptable disintegrants comprise starch, ion exchange resins, e.g. Amberlite, cross-linked polyvinylpyrrolidone, modified cellulose gum, e.g. croscarmellose sodium (e.g. Ac-di-Sol^{®}), sodium starch glycollate, sodium carboxymethylcellulose, sodium dodecyl sulphate, modified corn starch, microcrystalline cellulose, magnesium aluminium silicate, alginic acid, alginate, powdered cellulose.

Pharmaceutically acceptable lubricants comprise magnesium stearate, calcium stearate, stearic acid, talc, polyethylene glycol, sodium lauryl sulfate, magnesium lauryl sulphate.

Tablets of the present invention may in addition include other optional excipients such as, for example, flavors, sweeteners and colors.

Solid pharmaceutical compositions according to the present invention may comprise by weight based on the total weight of the composition :
(a) from 5 to 50% of a compound of formula (I), (I-a) or (I-b);
(b) from 0.01 to 5% of a wetting agent;
(c) from 40 to 92% of a diluent;
(d) from 0.1 to 5% of a glidant.

Tablets according to the present invention may comprise by weight based on the total weight of the tablet core :
(a) from 5 to 50% of a compound of formula (I), (I-a) or (I-b);
(b) from 0.01 to 5% of a wetting agent;
(c) from 40 to 92% of a diluent;
(d) from 0 to 10% of a polymer;
(e) from 2 to 10% of a disintegrant;
(f) from 0.1 to 5% of a glidant;
(g) from 0.1 to 1.5% of a lubricant.

Tablets of the present invention may optionally be film-coated following art-known coating procedures. Film-coated tablets are easier to swallow than uncoated tablet cores, are usually easier to distinguish from other tablets - in particular when the film-coat contains a dye or a pigment -, may have reduced tackiness, and may furthermore have an improved stability (increased shelf-life), e.g. because the coating may protect the active ingredient from the influence of light. Preferably, the film coat is an immediate release coat. Film coatings may comprise a film-forming polymer and optionally a plasticizer or a pigment. An example of a suitable film-forming polymer is hydroxypropyl methylcellulose, and an example of a suitable plasticizer is polyethyleneglycol, e.g. macrogol 3000 or 6000, or triacetin. Commercially available suitable coatings for pharmaceutical tablets are well-known to a person skilled in the art. Preferably, the film coating is a non-transparant film coating. An example of a suitable coating is Opadry^{®}, in particular coating powder Opadry^{®} II White.

Tablets of the present invention can be prepared by direct compression or wet granulation.

Therefore, the present invention is also concerned with a process of preparing a tablet comprising a compound of formula (I), (I-a) or (I-b) comprising the steps of :
(i) dry blending the active ingredient, the disintegrant and the optional glidant with the diluent;
(ii) optionally mixing the lubricant with the mixture obtained in step (i);
(iii) compressing the mixture obtained in step (i) or in step (ii) in the dry state into a tablet; and
(iv) optionally film-coating the tablet obtained in step (iii).

The present invention is also concerned with a process of preparing a tablet comprising a compound of formula (1), (I-a) or (I-b) comprising the steps of :
(i) dry blending the active ingredient and part of the diluent;
(ii) preparing a binder solution by dissolving the binder and the wetting agent in the binder solution solvent;
(iii) spraying the binder solution obtained in step (ii) on the mixture obtained in step (i);
(iv) drying the wet powder obtained in step (iii) followed by sieving and optionally mixing;
(v) mixing the remaining part of the diluent, the disintegrant and the optional glidant in the mixture obtained in step (iv);
(vi) optionally adding the lubricant to the mixture obtained in step (v);
(vii) compressing the mixture obtained in step (vi) into a tablet;
(viii) optionally film-coating the tablet obtained in step (vii).

A person skilled in the art will recognize the most appropriate equipment to be used for the above-described processes.

The above general route of preparing tablets of the present invention may be modified by a person skilled in the art by for instance adding certain ingredients at other stages than indicated above.

The present compound of formula (I), (I-a) or (I-b) can be used alone or in combination with other therapeutic agents, such as anti-virals, antibiotics, immunomodulators or vaccines for the treatment of viral infections. They may also be used alone or in combination with other prophylactic agents for the prevention of viral infections. The present compounds may be used in vaccines and methods for protecting individuals against viral infections over an extended period of time. The compounds may be employed in such vaccines either alone or together with other anti-viral agents in a manner consistent with the conventional utilization of reverse transcriptase inhibitors in vaccines. Thus, the present compounds may be combined with pharmaceutically acceptable adjuvants conventionally employed in vaccines and administered in prophylactically effective amounts to protect individuals over an extended period of time against HIV infection.

Also, the combination of an antiretroviral compound and a compound of formula (I), (I-a) or (I-b) can be used as a medicine. Thus, the present invention also relates to a product containing (a) a compound of formula (1), (I-a) or (I-b), and (b) one or more other antiretroviral compounds, as a combined preparation for simultaneous, separate or sequential use in anti-HIV treatment. In particular, the invention also relates to a product containing (a) a compound of formula (I), (I-a) or (I-b), and (b) one or more other antiretroviral compounds, as a combined preparation for simultaneous, separate or sequential use in anti-HIV treatment provided that the one or more other antiretroviral compounds are other than nucleoside reverse transcriptase inhibitors and/or nucleotide reverse transcriptase inhibitors. The different drugs may be combined in a single preparation together with pharmaceutically acceptable carriers. Thus, the present invention also relates to a pharmaceutical composition comprising a pharmaceutically acceptable carrier and (a) a therapeutically effective amount of a compound of formula (I), (I-a) or (I-b) and (b) one or more other antiretroviral agents.

Said other antiretroviral compounds may be known antiretroviral compounds such as suramine, pentamidine, thymopentin, castanospermine, dextran (dextran sulfate), foscarnet-sodium (trisodium phosphono formate); nucleoside reverse transcriptase inhibitors, e.g. zidovudine (3'-azido-3'-deoxythymidine, AZT), didanosine (2',3'-dideoxyinosine; ddI), zalcitabine (dideoxycytidine, ddC) or lamivudine (2'-3'-dideoxy-3'-thiacytidine, 3TC), stavudine (2',3'-didehydro-3'-deoxythymidine, d4T), abacavir, abacavir sulfate, emtricitabine ((-) FTC), racemic FTC and the like; non-nucleoside reverse transcriptase inhibitors such as nevirapine (11-cyclopropyl-5,11-dihydro-4-methyl-6*H*-dipyrido-[3,2-b : 2',3'-e][1,4]diazepin-6-one), efavirenz, delavirdine, TMC-120, TMC-125 and the like; compounds of the TIBO (tetrahydro-imidazo[4,5,1-jk][1,4]-benzodiazepine-2(1*H*)-one and thione)-type e.g. (S)-8-chloro-4,5,6,7-tetrahydro-5-methyl-6-(3-methyl-2-butenyl)imidazo-[4,5,1-jk][1,4]benzodiazepine-2(1*H*)-thione; compounds of the α-APA (α-anilino phenyl acetamide) type e.g. α-[(2-nitrophenyl)amino]-2,6-dichlorobenzene-acetamide and the like; inhibitors of trans-activating proteins, such as TAT-inhibitors, e.g. RO-5-3335, or REV inhibitors, and the like; protease inhibitors e.g. indinavir, ritonavir, saquinavir, lopinavir (ABT-378), nelfinavir, amprenavir, TMC-114, BMS-232632, VX-175 and the like; fusion inhibitors, e.g. T-20, T-1249 and the like; CXCR4 receptor antagonists, e.g. AMD-3100 and the like; inhibitors of the viral integrase; nucleotide-like reverse transcriptase inhibitors, e.g. tenofovir, tenofovir diphosphate, tenofovir disoproxil fumarate and the like; ribonucleotide reductase inhibitors, e.g. hydroxyurea and the like; CCR5 antagonists, e.g. ancriviroc, aplaviroc hydrochloride, vicriviroc.

By administering the compounds of the present invention with other anti-viral agents which target different events in the viral life cycle, the therapeutic effect of these compounds can be potentiated. Combination therapies as described above exert a synergistic effect in inhibiting HIV replication because each component of the combination acts on a different site of HIV replication. The use of such combinations may reduce the dosage of a given conventional anti-retroviral agent which would be required for a desired therapeutic or prophylactic effect as compared to when that agent is administered as a monotherapy. These combinations may reduce or eliminate the side effects of conventional single anti-retroviral therapy while not interfering with the anti-viral activity of the agents. These combinations reduce potential of resistance to single agent therapies, while minimizing any associated toxicity. These combinations may also increase the efficacy of the conventional agent without increasing the associated toxicity.

The compounds of the present invention may also be administered in combination with immunomodulating agents, e.g. levamisole, bropirimine, anti-human alpha interferon antibody, interferon alpha, interleukin 2, methionine enkephalin, diethyldithiocarbamate, tumor necrosis factor, naltrexone and the like; antibiotics, e.g. pentamidine isethiorate and the like; cholinergic agents, e.g. tacrine, rivastigmine, donepezil, galantamine and the like; NMDA channel blockers, e.g. memantine to prevent or combat infection and diseases or symptoms of diseases associated with HIV infections, such as AIDS and ARC, e.g. dementia.

Although the present invention focuses on the use of the present compounds for preventing or treating HIV infections, the present compounds may also be used as inhibitory agents for other viruses which depend on similar reverse transcriptases for obligatory events in their life cycle.

### Experimental part

### A. Synthesis of the compound of formula (I-a)

One mol of (E) 4-[[4-[[4-(2-cyanoethenyl)-2,6-dimethylphenyl]amino]-2-pyrimidinyl]amino]benzonitrile free base was dissolved in acetic acid (2L/mol at 80-100°C). 1.2 mol of fumaric acid was added.

At 60-70 °C, water (2L/mol) was added portionwise.

The mixture was stirred overnight at room temperature.

The precipitate was filtered, washed twice with water and dried in vacuo at 50 °C, yielding 90 % of a compound of formula (I-a).

### B. Solubility data

Table 1 lists solubility data of free base (E) 4-[[4-[[4-(2-cyanoethenyl)-2,6-dimethylphenyl]amino]-2-pyrimidinyl]amino]benzonitrile and of the compound of formula (I-a).

**Table 1 :**

| Compound | Concentration in mg/ml | | |
|---|---|---|---|
| | Water | 0.01 N HCl | PEG 400 |
| Free base (E-isomer) | 0.00002 | 0.019 | 40 |
| Compound of formula (I-a) | 0.0009 | 0.013 | |

The free base as well as the fumarate salt have a poor solubility in water as well as in 0.01 N HCl. Free base and fumarate salt may be classified as BCS class 2 compounds. The solubility of the free base is significantly increased in PEG 400.

### C. Stability data

### a) Chemical stability

Compound (I-a) was stored under different conditions of humidity and temperature. After storage, the salt was analyzed by High Performance Liquid Chromatography (HPLC) for percentage of impurities.

The results are gathered in Table 2 below. It can be concluded that the compound of formula (I-a) is chemically stable.

**Table 2:**

| **Storage condition** | **Sum of impurities % (%, w/w)** | | |
|---|---|---|---|
| | **1 week** | **4 weeks** | **8 weeks** |
| **Reference** | 0.58 | - | - |
| 40°C/75% RH | - | 0.62 | 0.61 |
| 50°C/air | - | 0.62 | 0.61 |
| RT/<5% RH | - | 0.61 | 0.62 |
| RT/56% RH | - | 0.58 | 0.64 |
| RT/75% RH | - | 0.59 | 0.65 |

| | | | |
|---|---|---|---|
| Explanatory note: - = not tested RT = room temperature RH = Relative Humidity | | | |

The compound of formula (I-a) was also found to be not hygroscopic.

### b) Physical stability

The stability of the crystal structure of the compound of formula (I-a) was studied after storage for a period of six weeks under different conditions of humidity and temperature. The same conditions as described in Table 2 were applied.

After storage the compound was analyzed with infrared spectroscopy.

No changes in crystal structure were observed, indicating that the compound is crystallographically stable.

### D. Tablet formulations

Tablet compositions illustrating the present invention are :

| Composition 1a | |
|---|---|
| *Tablet core :* | |
| Compound of formula (I-a) | 32.9 mg (i.e. 25 mg base equivalent) |
| Lactose monohydrate | 236.6 mg |
| Hypromellose 2910 15mPa.s | 5.6 mg |
| Polysorbate 20 | 1.4 mg |
| Microcrystalline cellulose | 52.5 mg |
| Croscarmellose sodium | 17.5 mg |
| Colloidal silicon dioxide | 1.05 mg |
| Magnesium stearate | 2.45 mg |
| | |
| *Tablet film coat* | |
| Coating powder Opadry^{®} II | White 14 mg |
| Purified water* | 80 µl |
| | |

| Composition 1b | |
|---|---|
| *Tablet core :* | |
| Compound of formula (I-a) | 32.9 mg (i.e. 25 mg base equivalent) |
| Lactose monohydrate | 46.85 mg |
| Hypromellose 2910 5mPa.s | 1.40 mg |
| Polysorbate 20 | 0.35 mg |
| Microcrystalline cellulose | 13.125 mg |
| Croscarmellose sodium | 4.375 mg |
| Magnesium stearate | 1.00 mg |
| | |
| *Tablet film coat* | |
| Coating powder Opadry^{®} II | White 4 mg |
| Purified water* | q.s. |
| | |

| Composition 1c | |
|---|---|
| *Tablet core :* | |
| Compound of formula (I-a) | 32.9 mg (i.e. 25 mg base equivalent) |
| Lactose monohydrate | 51.57 mg |
| Hypromellose 2910 5mPa.s | 1.75 mg |
| Polysorbate 20 | 0.35 mg |
| Silicified microcrystalline | cellulose 16.83 mg |
| Croscarmellose sodium | 5.5 mg |
| Magnesium stearate | 1.10 mg |
| *Tablet film coat* | |
| Coating powder Opadry^{®} II White | 4.4 mg |
| Purified water* | q.s. |
| | |

| Composition 1d | |
|---|---|
| *Tablet core :* | |
| Compound of formula (I-a) | 32.9 mg (i.e. 25 mg base equivalent) |
| Lactose monohydrate | 49.745 mg |
| Polyvinylpyrrolidone | 3.25 mg |
| Polysorbate 20 | 0.35 mg |
| Silicified microcrystalline cellulose | 16.605 mg |
| Croscarmellose sodium | 6.05 mg |
| Magnesium stearate | 1.10 mg |
| | |
| *Tablet film coat* | |
| Coating powder Opadry^{®} II White | 4.4 mg |
| Purified water* | q.s. |
| | |

| Composition 2a | |
|---|---|
| *Tablet core :* | |
| Compound of formula (I-a) | 110 mg (i.e. 100 mg base equivalent) |
| Lactose monohydrate | 137.8 mg |
| Hypromellose 2910 15mPa.s | 5.6 mg |
| Polysorbate 20 | 1.4 mg |
| Microcrystalline cellulose | 52.5 mg |
| Croscarmellose sodium | 17.5 mg |
| Colloidal silicon dioxide | 1.05 mg |
| Magnesium stearate | 2.45 mg |
| | |
| *Tablet film coat* | |
| Coating powder Opadry^{®} II White | 14 mg |
| Purified water* | 80 µl |
| | |

| Composition 2b | |
|---|---|
| *Tablet core :* | |
| Compound of formula (I-a) | 131.7 mg (i.e. 100 mg base equivalent) |
| Lactose monohydrate | 187.3 mg |
| Hypromellose 2910 5mPa.s | 5.6 mg |
| Polysorbate 20 | 1.4 mg |
| Microcrystalline cellulose | 52.5 mg |
| Croscarmellose sodium | 17.5 mg |
| Magnesium stearate | 4.00 mg |
| | |
| *Tablet film coat* | |
| Coating powder Opadry^{®} II White | 16 mg |
| Purified water* | q.s. |
| | |

| Composition 2c | |
|---|---|
| *Tablet core :* | |
| Compound of formula (I-a) | 131.7 mg (i.e. 100 mg base equivalent) |
| Lactose monohydrate | 206.18 mg |
| Hypromellose 2910 5mPa.s | 7.00 mg |
| Polysorbate 20 | 1.4 mg |
| Silicified microcrystalline cellulose | 67.32 mg |
| Croscarmellose sodium | 22.00 mg |
| Magnesium stearate | 4.40 mg |
| | |
| *Tablet film coat* | |
| Coating powder Opadry^{®} II White | 17.6 mg |
| Purified water* | q.s. |
| | |

| Composition 2d | |
|---|---|
| *Tablet core :* | |
| Compound of formula (I-a) | 131.7 mg (i.e. 100 mg base equivalent) |
| Lactose monohydrate | 198.88 mg |
| Polyvinylpyrrolidone | 13.00 mg |
| Polysorbate 20 | 1.4 mg |
| Silicified microcrystalline cellulose | 66.42 mg |
| Croscarmellose sodium | 24.2 mg |
| Magnesium stearate | 4.40 mg |
| | |
| *Tablet film coat* | |
| Coating powder Opadry^{®} II White | 17.6 mg |
| Purified water* | q.s. |

| Composition 3a | |
|---|---|
| *Tablet core :* | |
| Compound of formula (I-a) | 65.8 mg (i.e. 50 mg base equivalent) |
| Lactose monohydrate | 203.7 mg |
| Hypromellose 2910 15mPa.s | 5.6 mg |
| Polysorbate 20 | 1.4 mg |
| Microcrystalline cellulose | 52.5 mg |
| Croscarmellose sodium | 17.5 mg |
| Colloidal silicon dioxide | 1.05 mg |
| Magnesium stearate | 2.45 mg |
| | |
| *Tablet film coat* | |
| Coating powder Opadry^{®} II White | 14 mg |
| Purified water* | 80 µl |
| | |

| Composition 3b | |
|---|---|
| *Tablet core :* | |
| Compound of formula (I-a) | 65.8 mg (i.e. 50 mg base equivalent) |
| Lactose monohydrate | 93.7 mg |
| Hypromellose 2910 5mPa.s | 2.80 mg |
| Polysorbate 20 | 0.70 mg |
| Microcrystalline cellulose | 26.25 mg |
| Croscarmellose sodium | 8.75 mg |
| Magnesium stearate | 2.00 mg |
| | |
| *Tablet film coat* | |
| Coating powder Opadry^{®} II White | 8.00 mg |
| Purified water* | q.s. |
| | |

| Composition 3c | |
|---|---|
| *Tablet core :* | |
| Compound of formula (I-a) | 65.8 mg (i.e. 50 mg base equivalent) |
| Lactose monohydrate | 103.14 mg |
| Hypromellose 2910 5mPa.s | 3.50 mg |
| Polysorbate 20 | 0.70 mg |
| Silicified microcrystalline | cellulose 33.66 mg |
| Croscarmellose sodium | 11.0 mg |
| Magnesium stearate | 2.20 mg |
| | |
| *Tablet film coat* | |
| Coating powder Opadry^{®} II White | 8.80 mg |
| Purified water* | q.s. |
| | |

| Composition 3d | |
|---|---|
| *Tablet core :* | |
| Compound of formula (I-a) | 65.8 mg (i.e. 50 mg base equivalent) |
| Lactose monohydrate | 99.49 mg |
| Polyvinylpyrrolidone | 6.50 mg |
| Polysorbate 20 | 0.70 mg |
| Silicified microcrystalline cellulose | 33.21 mg |
| Croscarmellose sodium | 12.1 mg |
| Magnesium stearate | 2.20 mg |
| | |
| *Tablet film coat* | |
| Coating powder Opadry^{®} II White | 8.80 mg |
| Purified water* | q.s. |
| | |

| Composition 4 | |
|---|---|
| *Tablet core :* | |
| Compound of formula (I-a) | 98.7 mg (i.e. 75 mg base equivalent) |
| Lactose monohydrate | 149.235 mg |
| Polyvinylpyrrolidone | 9.75 mg |
| Polysorbate 20 | 1.05 mg |
| Silicified microcrystalline cellulose | 49.815 mg |
| Croscarmellose sodium | 18.15 mg |
| Magnesium stearate | 3.30 mg |
| | |
| *Tablet film coat* | |
| Coating powder Opadry^{®} II White | 13.2 mg |
| Purified water* | q.s. |

| Composition 5a | |
|---|---|
| *Tablet core :* | |
| Compound of formula (I-a) | 197.4 mg (i.e. 150 mg base equivalent) |
| Lactose monohydrate | 298.47 mg |
| Polyvinylpyrrolidone | 19.5 mg |
| Polysorbate 20 | 2.1 mg |
| Silicified microcrystalline cellulose | 99.63 mg |
| Croscarmellose sodium | 36.30 mg |
| Magnesium stearate | 6.6 mg |
| | |
| *Tablet film coat* | |
| Coating powder Opadry^{®} II White | 19.80 mg |
| Purified water* | q.s. |
| | |

| Composition 5b | |
|---|---|
| *Tablet core :* | |
| Compound of formula (I-a) | 197.4 mg (i.e. 150 mg base equivalent) |
| Lactose monohydrate | 309.42 mg |
| Hypromellose 2910 5 mPa.s | 10.5 mg |
| Polysorbate 20 | 2.1 mg |
| Silicified microcrystalline cellulose | 100.98 mg |
| Croscarmellose sodium | 33.00 mg |
| Magnesium stearate | 6.6 mg |
| | |
| *Tablet film coat* | |
| Coating powder Opadry^{®} II White | 19.80 mg |
| Purified water* | q.s. |

| | |
|---|---|
| * not present in final tablet | |

The above tablets are prepared by dissolving hypromellose or polyvinylpyrrolidone and polysorbate 20 in purified water (q.s.) followed by spraying said solution on fluidized powder consisting of a mixture of Form A and lactose monohydrate. The obtained granulate is dried, sieved and mixed with microcrystalline cellulose or silicified microcrystalline cellulose, croscarmellose sodium and optionally colloidal silicon dioxide. After addition of Magnesium stearate, the powder mixture is compressed into tablets followed by film coating the tablets with a suspension of Coating powder Opadry^{®} II White in purified water.

In the above compositions, microcrystalline cellulose is preferably Avicel^{®} PH101, croscarmellose sodium is preferably Ac-Di-Sol^{®}; silicified microcrystalline cellulose is preferably Prosolv^{®}HD90; polyvinylpyrrolidone is preferably PVP K29-32.

### E. In vivo bioavailability study

The *in vivo* bioavailability of the compound of formula (I-a) was studied in male beagle dogs.

The formulations used for oral administration were :
- a PEG 400 solution of (E) 4-[[4-[[4-(2-cyanoethenyl)-2,6-dimethylphenyl]amino]-2-pyrimidinyl]amino]benzonitrile free base (25 mg/ml) (group I);
- a capsule (size 0; red cap-red body) containing a mixture consisting of 32.9 mg of compound of formula (I) (i.e. 25 mg base equivalent); 300 mg lactose DC (direct compression); 0.59 mg of silicon dioxide; 0.59 mg of sodium lauryl sulphate (group II);

The formulations of group II were orally administered at a dose level of 5 mg base equivalent/kg. The formulations were prepared based on previously determined body weights of the animals. The exact administered dose was calculated using the body weights just before dosing and amounted on average to 5 mg base equivalent/kg.

The reference PEG400 formulation (group I) was administered orally via gavage by use of a stomach tube at a daily volume of 0.2 ml/kg body weight The stomach tube was flushed with 2 ml of PEG400 per dog, followed by the placement of a syringe of 10 ml filled with air on the stomach tube. The tube was removed after a pause of 10 to 15 seconds.

The reference PEG400 solution (group I) and the compound of formula (I-a) (group II) were dosed according to a cross-over design. The first group of 2 dogs was dosed with the reference formulation of group I at 5 mg eq./kg (0.2 ml/kg) and the second group of 2 dogs was dosed with the fumarate salt formulation of group II at 5 mg basd eq./kg (2 capsules/dog). After a washout-period of 14 days, the first group of dogs was dosed with the fumarate salt (group II) and the second group with the reference formulation (group I).

Blood samples (3 ml on EDTA) were taken from a jugular vein at 0 (= predose), 0.5, 1, 2, 4, 6, 8, 24, 32, 48, 72 and 96 hours after dosing on day 0 and day 14. Immediately after blood sampling, blood samples were shielded from light.

Blood samples were centrifuged at room temperature at 1900 x g for about 10 minutes to allow plasma separation. Plasma was separated, transferred into a second tube, and frozen within two hours of blood sampling.

Plasma samples were analysed individually for (E)-4-[[4-[[4-(2-cyanoethenyl)-2,6-dimethylphenyl]amino]-2-pyrimidinyl]amino]benzonitrile by means of a validated LC-MS/MS method.

LC-MS/MS analysis was carried out on an API-3000 system (Applied Biosystems), which was coupled to an HPLC-system (Agilent).

Individual plasma concentration-time profiles were subjected to a non-compartmental pharmacokinetic analysis using WinNonlin software (WinNonlin Release 4.0.1a Enterprise, Pharsight Corporation, Mountain View, California, U.S.A.). Peak plasma concentrations (Cₘₐₓ) and corresponding peak times (Tₘₐₓ) were calculated. The area under the plasma concentration-time curve (AUC₀₋ₜ) was calculated using the linear up/log down trapezoidal rule. The AUC_{0-∞} was calculated as the sum of AUC₀₋₉₆ₕ and C₉₆ₕ/β, with β, the elimination rate constant, determined by log-linear regression of the terminal plasma concentration-time data. Mean plasma concentrations and mean pharmacokinetic parameters were calculated per formulation.

Mean plasma concentrations and basic pharmacokinetic parameters of group I and II are given in Table 3.

**Table 3: Mean (± S.D) plasma concentrations together with some basic pharmacokinetic parameters of (E)-4-[[4-[[4-(2-cyanoethenyl)-2,6-dimethylphenyl]amino]-2-pyrimidinyl]amino]benzonitrile in male beagle dogs after oral administration of formulation of group I and II dosed at 5 mg base eq./kg.**

| **Time (h)** | | **Group I** | **Group II** |
|---|---|---|---|
| **0** | | <1.0 | <1.0 |
| **0.5** | | 104 ± 79 | 5.64 ± 2.76 |
| **1** | | 258 ± 79 | 108 ± 75 |
| **2** | | 478 ± 83 | 362 ± 132 |
| **4** | | 462 ± 155 | 372 ± 121 |
| **6** | | 354 ± 111 | 346 ± 141 |
| **8** | | 256 ± 117 | 247 ± 94 |
| **24** | | 179 ± 106 | 187 ± 50 |
| **32** | | 150 ± 89 | 163 ± 66 |
| **48** | | 79.4 ± 49.1 | 89.2 ± 39.7 |
| **72** | | 42.5 ± 25.1 | 50.9 ± 29.7 |
| **96** | | 21.6 ± 15.6 | 25.0 ± 12.6 |
| **Cₘₐₓ** | (ng/ml) | 523 ± 104 | 417 ± 125 |
| **Tₘₐₓ** | (h) | 3.0 ± 1.2 | 4.0 ± 2.3 |
| **AUC₀₋₇₂** | (ng.h/ml) | 11497 ± 5437 | 11527 ± 2896 |
| **AUC_{0-inf}** | (ng.h/ml) | 12299 ± 5906 | 12489 ± 3405 |

Based on the AUC-values, the fumarate salt capsule formulation seems bioequivalent to the reference PEG400 solution of (E)-4-[[4-[[4-(2-cyanoethenyl)-2,6-dimethylphenyl]amino]-2-pyrimidinyl]amino]benzonitrile.

## Claims

1. A solid pharmaceutical composition for oral administration comprising a pharmaceutically acceptable carrier and as active ingredient a therapeutically effective amount of a compound of formula (I) 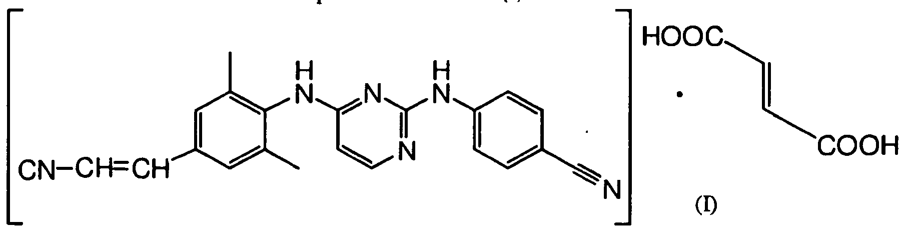 a N-oxide or a stereochemically isomeric form thereof, and further comprising a wetting agent.

2. A pharmaceutical composition according to claim 1 wherein the compound of formula (I) is a compound of formula (I-a)

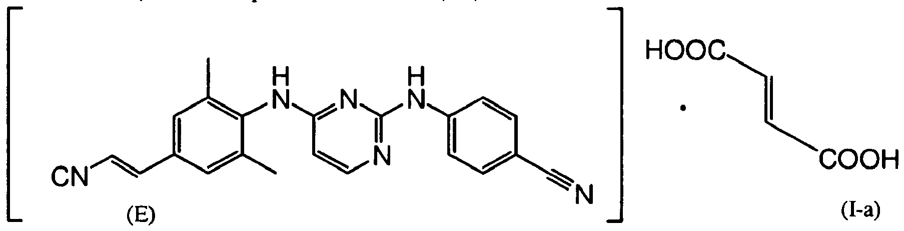

3. A pharmaceutical composition according to claim 1 or 2 wherein the wetting agent is Tween.

4. A pharmaceutical composition according to any one of claims 1 to 3 comprising by weight based on the total weight of the composition :
(a) from 5 to 50% of active ingredient;
(b) from 0.01 to 5 % of a wetting agent;
(c) from 40 to 92% of a diluent;
(d) from 0.1 to 5% of a glidant.

5. A pharmaceutical composition according to any one of the preceding claims wherein the composition is in the form of a tablet.

6. A pharmaceutical composition according to claim 5 which is film-coated.

7. A pharmaceutical composition according to claim 5 having the following composition by weight based on the total weight of the tablet core
(a) from 5 to 50% of active ingredient;
(b) from 0.01 to 5 % of a wetting agent;
(c) from 40 to 92% of a diluent;
(d) from 0 to 10 % of a polymer;
(e) from 2 to 10 % of a disintegrant;
(f) from 0.1 to 5% of a glidant;
(g) from 0.1 to 1.5 % of a lubricant.

8. Use of a pharmaceutical composition as claimed in any one of the preceding claims for the manufacture of a medicament for the treatment or the prevention of HIV infection.

## Patentansprüche

1. Feste pharmazeutische Zusammensetzung zur oralen Verabreichung, umfassend einen pharmazeutisch unbedenklichen Träger und als Wirkstoff eine therapeutisch wirksame Menge einer Verbindung der Formel (I) 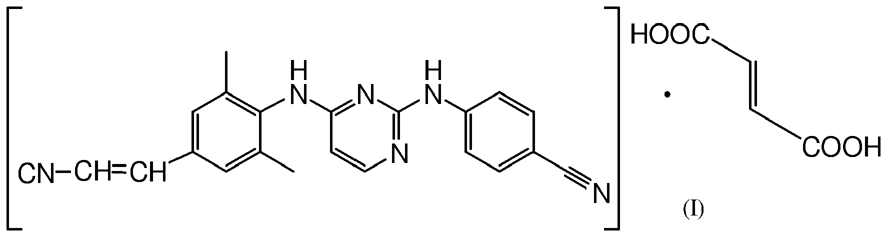 eines N-Oxids oder einer stereochemisch isomeren Form davon und ferner ein Netzmittel.

2. Pharmazeutische Zusammensetzung nach Anspruch 1, wobei es sich bei der Verbindung der Formel (I) um eine Verbindung der Formel (I-a) 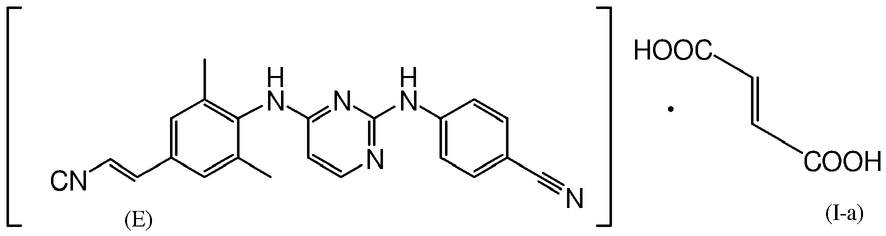 handelt.

3. Pharmazeutische Zusammensetzung nach Anspruch 1 oder 2, wobei es sich bei dem Netzmittel um Tween handelt.

4. Pharmazeutische Zusammensetzung nach einem der Ansprüche 1 bis 3, umfassend, bezogen auf das Gesamtgewicht der Zusammensetzung:
(a) 5 bis 50 Gew.-% Wirkstoff;
(b) 0,01 bis 5 Gew.-% Netzmittel;
(c) 40 bis 92 Gew.-% Verdünnungsmittel;
(d) 0,1 bis 5 Gew.-% Gleitmittel.

5. Pharmazeutische Zusammensetzung nach einem der vorhergehenden Ansprüche, wobei die Zusammensetzung in Form einer Tablette vorliegt.

6. Pharmazeutische Zusammensetzung nach Anspruch 5 mit Filmüberzug.

7. Pharmazeutische Zusammensetzung nach Anspruch 5 mit der folgenden Zusammensetzung, bezogen auf das Gesamtgewicht des Tablettenkerns:
(a) 5 bis 50 Gew.-% Wirkstoff;
(b) 0,01 bis 5 Gew.-% Netzmittel;
(c) 40 bis 92 Gew.-% Verdünnungsmittel;
(d) 0 bis 10 Gew.-% Polymer;
(e) 2 bis 10 Gew.-% Sprengmittel;
(f) 0,1 bis 5 Gew.-% Gleitmittel;
(g) 0,1 bis 1,5 Gew.-% Schmiermittel.

8. Verwendung einer pharmazeutischen Zusammensetzung gemäß einem der vorhergehenden Ansprüche zur Herstellung eines Arzneimittels zur Behandlung oder Prävention von HIV-Infektion.

## Revendications

1. Composition pharmaceutique solide destinée à une administration orale, comprenant un véhicule pharmaceutiquement acceptable et, comme ingrédient actif, une quantité thérapeutiquement efficace d'un composé de formule (I) 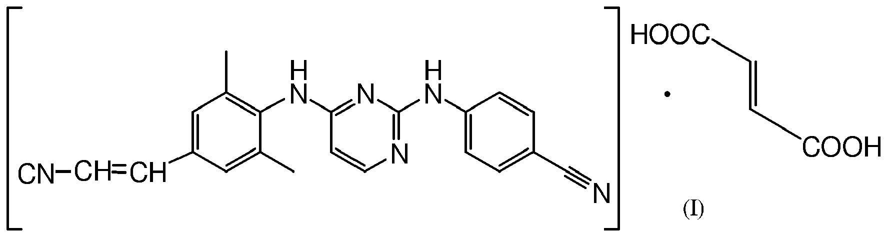 d'un N-oxyde ou d'une forme stéréochimiquement isomère de celui-ci, et comprenant en outre un agent mouillant.

2. Composition pharmaceutique selon la revendication 1, dans laquelle le composé de formule (I) est un composé de formule (I-a)

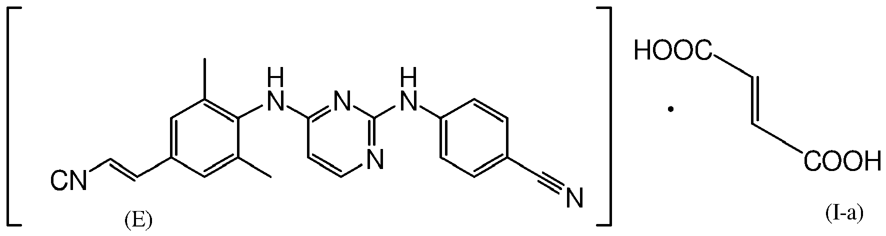

3. Composition pharmaceutique selon la revendication 1 ou 2, dans laquelle l'agent mouillant est le Tween.

4. Composition pharmaceutique selon l'une quelconque des revendications 1 à 3, comprenant, en poids sur la base du poids total de la composition :
(a) de 5 à 50% d'ingrédient actif ;
(b) de 0,01 à 5% d'un agent mouillant ;
(c) de 40 à 92% d'un diluant ;
(d) de 0,1 à 5% d'un agent glissant.

5. Composition pharmaceutique selon l'une quelconque des revendications précédentes, où la composition se trouve sous la forme d'un comprimé.

6. Composition pharmaceutique selon la revendication 5, qui est enrobée d'un film.

7. Composition pharmaceutique selon la revendication 5, ayant la composition suivante, en poids sur la base du poids total du coeur de comprimé :
(a) de 5 à 50% d'ingrédient actif ;
(b) de 0,01 à 5% d'un agent mouillant ;
(c) de 40 à 92% d'un diluant ;
(d) de 0 à 10% d'un polymère ;
(e) de 2 à 10% d'un agent délitant ;
(f) de 0,1 à 5% d'un agent glissant ;
(g) de 0,1 à 1,5% d'un lubrifiant.

8. Utilisation d'une composition pharmaceutique selon l'une quelconque des revendications précédentes, pour l'élaboration d'un médicament destiné au traitement ou à la prévention d'une infection par le VIH.
